# EUROPEAN PATENT SPECIFICATION

(11) **EP 3 057 484 B1**
(45) Date of publication and mention of the grant of the patent: **27.03.2019**
(21) Application number: 14780637.6
(22) Date of filing: 23.09.2014
(51) Int. Cl.: A61B 1/12, A61B 90/70

(54) **MODULAR DRYING DEVICE FOR ENDOSCOPES**
MODULARE TROCKNUNGSVORRICHTUNG FÜR ENDOSKOPE
DISPOSITIF MODULAIRE DE SÉCHAGE POUR ENDOSCOPES

(30) Priority: 14.10.2013 NL 2011604
(43) Date of publication of application: 24.08.2016
(73) Proprietor: Van Vliet Medical Supply B.V., 1322 AJ Almere (NL)
(72) Inventor: HAZE, Jacob, NL-3766 HT Soest (NL)
(74) Representative: Klavers, Cornelis
(86) International application number: PCT/NL2014/050651
(87) International publication number: WO 2015/057057

(56) References cited:
- EP-A2- 0 835 665
- WO-A2-2010/136093
- DE-A1- 10 125 521
- US-A- 5 425 815
- US-A- 5 961 937
- US-A1- 2009 217 956
- US-A1- 2012 139 398

## Description

The present invention relates to a drying device for one or more endoscopes, comprising a drying space for said one or more endoscopes.

Such a drying device for one or more endoscopes is known from US-2012/139398 A1 and comprises a drying space comprising holder means for hanging the endoscopes, a mounting unit situated at the top of the drying space and provided with an air pressure chamber which is connected to blowing elements which open into the drying space and which are arranged to bring about a downward airflow in the drying space. The blowing elements may comprise various types of blowing elements, such as blowing elements of a type which bring about a downward airflow along and/or under the holder means, and close to the holder means, an air connection for the endoscope.

It is an object of the present invention to provide a more efficient, in particular externally drying, drying device.

To achieve this, the drying device according to the invention is characterised by blowing elements of a first type which bring about a downward airflow along the rear wall of the drying space, while the blowing elements of the second type bring about said downward airflow along and/or under the holder means, and in that the drying device comprises upstream of said air connection, an airflow meter which is intended for each individual endoscope, and which is used to measure a dry airflow through the interior of the endoscope.

An advantage of the drying device according to the invention resides in that the holder means ensure that the endoscopes are hung vertically downward, so that internal as well as external moisture flows in a downward direction. It is further advantageous that at least the external stream of moisture is stimulated to move in a downward direction by the airflow which, by way of the blowing elements, flows from the mounting unit in a downward direction in the drying space and also around the hanging endoscopes.

The effect of the combined action of both types of blowing elements is that a uniform downward airflow through the drying device is generated and maintained, which moves around all hanging endoscopes. As a result, independent of the actual position of the individual endoscopes in the drying device, a constant, reproducible drying capacity and drying quality is achieved.

The airflow meter is intended for each individual endoscope and is used to measure the dry airflow through the interior of the endoscope. Preferred embodiments of the invention are defined in the dependent claims.

A further embodiment of the drying device, which is advantageous from a construction-technical point of view, is characterized in that the mounting unit is a ceiling module in or of the drying space.

Mechanically and functionally required parts of the drying device, as mentioned hereinbefore and hereinafter, can be built into the ceiling and provided on the ceiling, if necessary prior to the assembly operation. By virtue of this, the construction, finishing and connection processes are performed in less time and without causing much inconvenience in situ.

Further detailed, possible embodiments, which are set forth in the remaining claims, are mentioned together with the associated advantages in the following description.

The drying device according to the invention will now be explained in greater detail with reference to the figures mentioned below, in which corresponding parts are indicated by means of the same reference numerals. In the Figures:
Figure 1 is a diagrammatic representation of a drying device in which a number of endoscopes hang to dry; and
Figure 2 is a diagrammatic representation of two drying devices which are separated from each other by a technical module, in which a splitting member connected to an air pump is situated which supplies air to both drying devices;
Figure 3 is a diagrammatic representation of the way in which airflows in the drying devices shown in figures 1 and 2 are influenced;
Figure 4 shows a detail of figure 3 which relates to a suspension indicator for an endoscope; and
Figure 5 shows an example of an airflow indicator which is used to measure the supply of air to and/or per individual endoscope suspension location in a respective drying device.

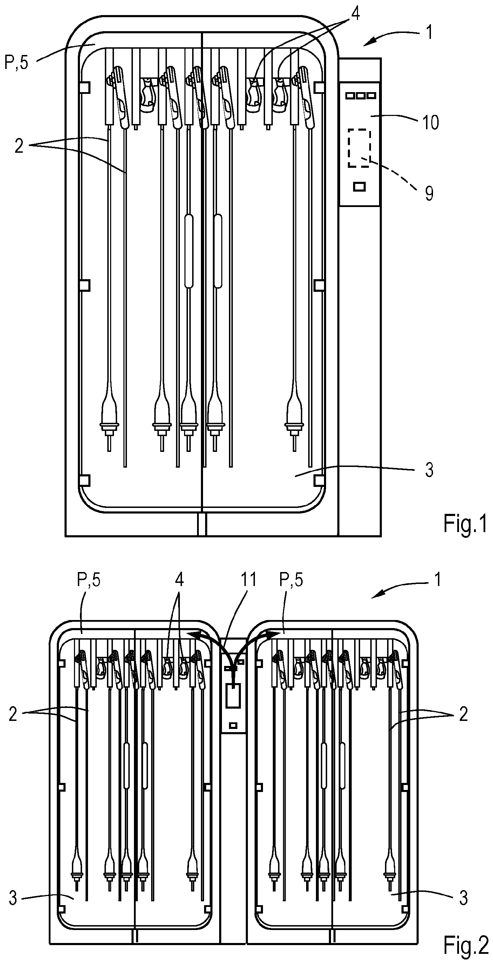

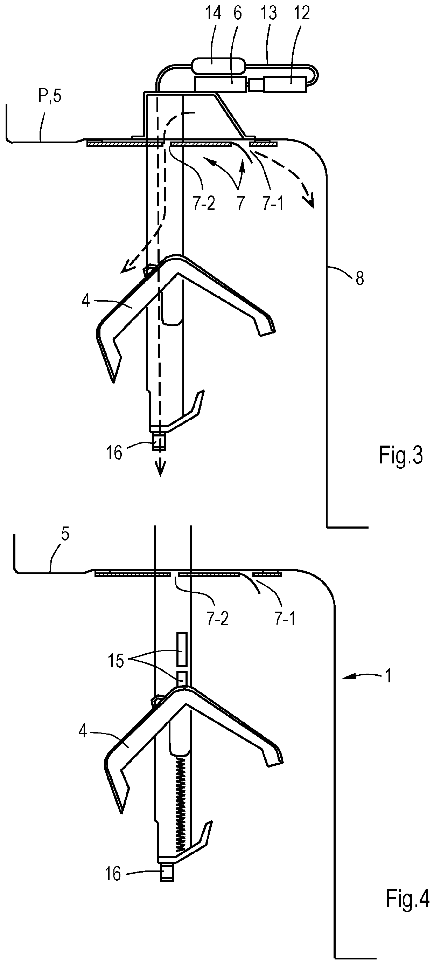

Figures 1 and 2 show, respectively, a drying device 1 which can be closed by means of access doors and a drying system comprising two drying devices 1 installed so as to be juxtaposed, and in each of which one or more endoscopes 2 can be hung to dry. The device 1 comprises a drying space 3 in which holder means 4 are provided for hanging the endoscopes 2. At the top of the drying space 3 there is situated a mounting unit 5 with an air pressure chamber 6 which is connected to blowing elements 7 which open into the drying space 3 and which are arranged to bring about a downward airflow in the drying space 3. There are two types of blowing elements 7, i.e. 7-1 and 7-2, which can be provided separately or, if necessary, together on the underside of the mounting unit 5 which is preferably the ceiling P of the space 3. The blowing elements 7-1 of the first type emit a downward airflow which passes around the endoscopes 2 and along the rear wall 8 of the drying space 3, such as indicated by means of a curved arrow in the diagrammatic representation of figure 3. The blowing elements 7-2 of the second type bring about a downward airflow through the drying space which passes along and/or under the holder means 4 and/or around the endoscopes 2, as indicated by means of straight arrows in figure 3. The blowing elements 7-1, 7-2 receive air, which is to be blown through the space 3, from the air pressure chamber 6 which is connected to an air pump 9 which is preferably arranged as high as possible, to minimize air pressure loss, in a technical module 10 on the side of the drying device 1.

In the system of figure 2, the technical module 10 is situated between the two drying devices 1, so that both air pressure chambers 6 accommodated therein receive fresh air by means of only one air pump 9 and via an airflow splitter 11 connected to the chambers 6. This is advantageous, inter alia, in terms of occupied space if in the course of time a second drying device has to be installed next to the first one, which second drying device does not have a -costly-module of its own. The air in the chamber(s) 6 is under sufficient pressure to ensure that, with or without the assistance of a cooperating fan in the module 10, a uniform airflow flowing around the endoscopes from top to bottom is maintained, so that at least the outside of the hanging endoscopes gets dry or remains dry. To this end, the air pressure is measured, if required, at various locations particularly inside the air pressure chamber 6 by means of air pressure sensors 12.

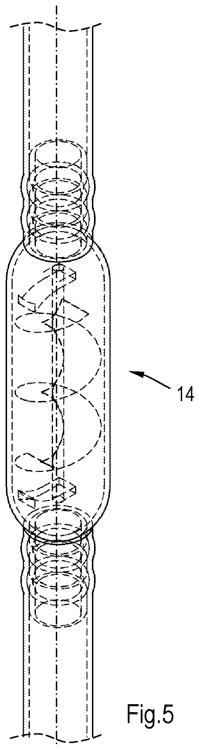

As shown in the diagrammatic representation of figure 3, the drying device 1 comprises an airflow meter 14, incorporated in the air pipe 13 to the pressure chamber 6 thereof, which airflow meter is schematically shown in Figure 5 and which is used to measure the current airflow quantities through the various blowing elements.

As is shown in figure 4, the holder means 4 intended for individually spring suspending each endoscope are secured via a magnetic contact 15, being a reed contact which is fixedly secured to the underside of the mounting unit 5. This results in an indication whether or not the endoscope hangs on the springing holder means 4 provided with a magnet.

In addition, the drying device 1 comprises, close to the holder means 4, a (compressed-)air connection 16 for the endoscope 1 and, if necessary, upstream of said (compressed-)air connection, an airflow meter which is intended for each individual endoscope and which is used to measure the dry airflow through the interior of the endoscope 2.

Furthermore, the drying device 1 comprises excess pressure means, not shown, which open into the drying space 3 and which are used to maintain, at least during the drying process, an air excess pressure in the drying space 3.

Said meter 14, said contact 15, an electric door fastening mounted on the access doors and the device 1, said excess pressure means and said sensors 12 are connected, together with indicator lighting which resembles a traffic light, to a processor-controlled data processing unit. In this manner, the programmable drying process is controlled, and the indication gives an insight into the status of the drying process from outside the device 1.

The drying device 1 is preferably composed of modules which can be easily assembled, i.e. the ceiling module P,5, the closable casing around the drying space 3 and the technical module 10 arranged next to the drying space.

## Claims

1. A drying device (1) for one or more endoscopes (2), which comprises;
- a drying space (3) comprising holder means (4) for hanging the endoscopes (2),
- a mounting unit (5) situated at the top of the drying space (3) and provided with an air pressure chamber (6) which is connected to blowing elements (7; 7-1, 7-2) which open into the drying space (3) and which are arranged to bring about a downward airflow in the drying space (3), which blowing elements (7; 7-1, 7-2) comprise various types of blowing elements, namely blowing elements of the type (7-2) which bring about a downward airflow along and/or under the holder means (4), and
- close to the holder means (4), an air connection (16) for the endoscope (2),
**characterised**
**by** blowing elements of a first type (7-1) which bring about a downward airflow along the rear wall (8) of the drying space (3), while the blowing elements of the second type (7-2) bring about said downward airflow along and/or under the holder means (4), and
in that the drying device (1) comprises upstream of said air connection (16), an airflow meter (14) which is intended for each individual endoscope (2), and which is used to measure a dry airflow through the interior of the endoscope (2).

2. The drying device (1) according to claim 1, **characterized in that** the drying space (3) is provided with an air pump (9) connected to the air pressure chamber (6), with an air pressure sensor (12) in the air pressure chamber which, by maintaining an excess pressure therein, maintains a uniform downward airflow through the drying space (3).

3. The drying device (1) according to claim 1 or 2, **characterized in that** the drying device (1) is composed of modules which are to be assembled, i.e. the drying space (3) which is provided with at least one access door on the front side, the technical module (10) arranged next to the drying space (3) and, if necessary, the ceiling module (P, 5) .

4. The drying device (1) according to any one of claims 1 to 3, **characterized in that** the drying device (1) comprises an airflow meter (14) incorporated in an air pipe (13) to the pressure chamber (6) thereof.

5. The drying device (1) according to any one of claims 1 to 4, **characterized in that** the holder means (4) intended for hanging an endoscope (2) are secured via a magnetic contact (15) to the underside of the mounting unit (5).

6. The drying device (1) according to claim 5, **characterized in that** the magnetic contact is a reed contact (15).

7. The drying device (1) according to any one of claims 1 to 6, **characterized in that**, for each endoscope (2), lighting is fitted to the mounting unit (5) in the drying space (3), which lighting indicates the progress of the processor controlled drying process of the respective endoscope (2).

8. The drying device (1) according to any one of claims 1 to 7, **characterized in that** the drying device (1) comprises excess pressure means which open into the drying space (3) and which are used to maintain, at least during the drying process, an air excess pressure in the drying space (3).

9. A drying system comprising at least one drying device (1) according to any one of claims 1 to 8, **characterized in that** a second drying device (1) arranged next to a first drying device (1) comprises a mounting unit (5) with an air pressure chamber (3) which, together with the air pressure chamber (6) of the first drying device (1), is connected via an airflow splitting member (11) to an air pump (9).

10. The drying system according to claim 9, **characterized in that** the mounting unit (5) is embodied by a ceiling in or of the drying space (3) of the second drying device (1).

## Patentansprüche

1. Trocknungsvorrichtung (1) für ein oder mehrere Endoskope (2), die Folgendes umfasst:
- einen Trocknungsraum (3), der Haltermittel (4) zum Aufhängen der Endoskope (2) umfasst,
- eine Anbringungseinheit (5), die am Oberteil des Trocknungsraums (3) angeordnet und mit einer Luftdruckkammer (6) versehen ist, die mit Blaselementen (7; 7-1, 7-2) verbunden ist, die sich in den Trocknungsraum (3) öffnen und die dafür angeordnet sind, einen Luftstrom nach unten in dem Trocknungsraum (3) zu veranlassen, wobei die Blaselemente (7; 7-1, 7-2) verschiedene Typen von Blaselementen umfassen, namentlich Blaselemente des Typs (7-2), die einen Luftstrom nach unten entlang der Haltermittel (4) und/oder unter denselben veranlassen, und
- nahe den Haltermittel (4), eine Luftverbindung (16) für das Endoskop (2),
**gekennzeichnet**
**durch** Blaselemente eines ersten Typs (7-1), die einen Luftstrom nach unten entlang der hinteren Wand (8) des Trocknungsraums (3) veranlassen, während die Blaselemente des zweiten Typs (7-2) den Luftstrom nach unten entlang der Haltermittel (4) und/oder unter denselben veranlassen, und
**dadurch**, dass die Trocknungsvorrichtung (1), stromaufwärts von der Luftverbindung (16), ein Luftdurchfluss-Messgerät (14) umfasst, das für jedes einzelne Endoskop (2) vorgesehen ist und das verwendet wird, um einen trockenen Luftstrom durch das Innere des Endoskops (2) zu messen.

2. Trocknungsvorrichtung (1) nach Anspruch 1, **dadurch gekennzeichnet, dass** der Trocknungsraum (3) mit einer Luftpumpe (9) versehen ist, die mit der Luftdruckkammer (6) verbunden ist, mit einem Luftdrucksensor (12) in der Luftdruckkammer, die, durch Aufrechterhalten eines Überdrucks in derselben, einen gleichförmigen Luftstrom nach unten durch den Trocknungsraum (3) aufrechterhält.

3. Trocknungsvorrichtung (1) nach Anspruch 1 oder 2, **dadurch gekennzeichnet, dass** die Trocknungsvorrichtung (1) aus Modulen zusammengestellt ist, die zusammengebaut werden müssen, d.h., dem Trocknungsraum (3), der mit wenigstens einer Zugangstür auf der vorderen Seite versehen ist, dem technischen Modul (10), das neben dem Trocknungsraum (3) angeordnet ist, und, falls notwendig, dem Deckenmodul (P, 5).

4. Trocknungsvorrichtung (1) nach einem der Ansprüche 1 bis 3, **dadurch gekennzeichnet, dass** die Trocknungsvorrichtung (1) ein Luftdurchfluss-Messgerät (14) umfasst, das in einem Luftrohr (13) zu der Druckkammer (6) derselben eingebaut ist.

5. Trocknungsvorrichtung (1) nach einem der Ansprüche 1 bis 4, **dadurch gekennzeichnet, dass** die Haltermittel (4) zum Aufhängen eines Endoskops (2) über einen magnetischen Kontakt (15) an der Unterseite der Anbringungseinheit (5) befestigt sind.

6. Trocknungsvorrichtung (1) nach Anspruch 5, **dadurch gekennzeichnet, dass** der magnetische Kontakt ein Zungenkontakt (15) ist.

7. Trocknungsvorrichtung (1) nach einem der Ansprüche 1 bis 6, **dadurch gekennzeichnet, dass**, für jedes Endoskop (2), eine Beleuchtung an der Anbringungseinheit (5) in dem Trocknungsraum (3) angebracht ist, wobei die Beleuchtung den Fortschritt des prozessorgesteuerten Trocknungsprozesses des jeweiligen Endoskops (2) anzeigt.

8. Trocknungsvorrichtung (1) nach einem der Ansprüche 1 bis 7, **dadurch gekennzeichnet, dass** die Trocknungsvorrichtung (1) Überdruckmittel umfasst, die sich in den Trocknungsraum (3) öffnen und die verwendet werden, um, wenigstens während des Trocknungsprozesses, einen Luftüberdruck in dem Trocknungsraum (3) aufrechtzuerhalten.

9. Trocknungsanlage, die wenigstens eine Trocknungsvorrichtung (1) nach einem der Ansprüche 1 bis 8 umfasst, **dadurch gekennzeichnet, dass** eine zweite Trocknungsvorrichtung (1), die neben einer ersten Trocknungsvorrichtung (1) angeordnet ist, eine Anbringungseinheit (5) mit einer Luftdruckkammer (3) umfasst, die, zusammen mit der Luftdruckkammer (6) der ersten Trocknungsvorrichtung (1), über ein Luftstrom-Teilungselement (11) mit einer Luftpumpe (9) verbunden ist.

10. Trocknungsanlage nach Anspruch 9, **dadurch gekennzeichnet, dass** die Anbringungseinheit (5) durch eine Decke in oder von dem Trocknungsraum (3) der zweiten Trocknungsvorrichtung (1) ausgeführt ist.

## Revendications

1. Dispositif de séchage (1) pour un ou plusieurs endoscopes (2), qui comprend :
- un espace de séchage (3) comprenant un moyen de retenue (4) pour accrocher les endoscopes (2),
- une unité de montage (5) située sur le dessus de l'espace de séchage (3) et pourvue d'une chambre de pression d'air (6) qui est connectée à des éléments soufflants (7; 7-1, 7-2) qui s'ouvrent sur l'espace de séchage (3) et qui sont conçus pour provoquer un flux d'air descendant dans l'espace de séchage (3), lesquels éléments soufflants (7; 7-1, 7-2) comprennent divers types d'éléments soufflants, à savoir des éléments soufflants du type (7-2) qui provoquent un flux d'air descendant le long et/ou sous le moyen de retenue (4), et
- près du moyen de retenue (4), une connexion d'air (16) pour l'endoscope (2),
**caractérisé**
**par** des éléments soufflants d'un premier type (7-1) qui provoquent un flux d'air descendant le long de la paroi arrière (8) de l'espace de séchage (3), alors que les éléments soufflants du second type (7-2) provoquent ledit flux d'air descendant le long et/sous le moyen de retenue (4), et
en ce que le dispositif de séchage (1) comprend, en amont de ladite connexion d'air (16), un mesureur de flux d'air (14) qui est destiné à chaque endoscope individuel (2) et qui est utilisé pour mesurer un flux d'air sec à travers l'intérieur de l'endoscope (2).

2. Dispositif de séchage (1) selon la revendication 1, **caractérisé en ce que** l'espace de séchage (3) est équipé d'une pompe à air (9) connectée à la chambre de pression d'air (6), sachant qu'il y a dans la chambre de pression d'air un capteur de pression d'air (12) qui, en maintenant une pression excédentaire à l'intérieur, maintient un flux d'air descendant uniforme à travers l'espace de séchage (3).

3. Dispositif de séchage (1) selon la revendication 1 ou 2, **caractérisé en ce que** le dispositif de séchage (1) est composé de modules qui sont assemblés, à savoir l'espace de séchage (3) qui est pourvu d'au moins une porte d'accès sur sa face avant, le module technique (10) disposé près de l'espace de séchage (3) et, si nécessaire, le module de plafond (P, 5).

4. Dispositif de séchage (1) selon l'une quelconque des revendications 1 à 3, **caractérisé en ce que** le dispositif de séchage (1) comprend un mesureur de flux d'air (14) incorporé dans une conduite à air (13) à la chambre de pression (6).

5. Dispositif de séchage (1) selon l'une quelconque des revendications 1 à 4, **caractérisé en ce que** le moyen de retenue (4) destiné à accrocher un endoscope (2) est fixé par l'intermédiaire d'un contact magnétique (15) à la face inférieure de l'unité de montage (5).

6. Dispositif de séchage (1) selon la revendication 5, **caractérisé en ce que** le contact magnétique est un contact Reed (15).

7. Dispositif de séchage (1) selon l'une quelconque des revendications 1 à 6, **caractérisé en ce que**, pour chaque endoscope (2), un voyant est fixé à l'unité de montage (5) dans l'espace de séchage (3), lequel voyant indique la progression du processus de séchage contrôlé par processeur de l'endoscope respectif (2).

8. Dispositif de séchage (1) selon l'une quelconque des revendications 1 à 7, **caractérisé en ce que** le dispositif de séchage (1) comprend un moyen de pression excédentaire qui s'ouvre dans l'espace de séchage (3) et qui est utilisé pour maintenir, au moins pendant le processus de séchage, une pression d'air excédentaire dans l'espace de séchage (3).

9. Système de séchage comprenant au moins un dispositif de séchage (1) selon l'une quelconque des revendications 1 à 8, **caractérisé en ce qu'**un second dispositif de séchage (1) disposé à proximité d'un premier dispositif de séchage (1) comprend une unité de montage (5) dotée d'une chambre de pression d'air (3) qui, avec la chambre de pression d'air (6) du premier dispositif de séchage (1), est connectée par l'intermédiaire d'un moyen de fractionnement de flux d'air (11) à une pompe à air (9).

10. Système de séchage selon la revendication 9, **caractérisé en ce que** l'unité de montage (5) est concrétisée par un plafond dans un ou de l'espace de séchage (3) du second dispositif de séchage (1).
